# EUROPEAN PATENT APPLICATION

(11) **EP 4 846 927 A1**
(43) Date of publication of application: **02.12.2026**
(21) Application number: 25209893.4
(22) Date of filing: 20.10.2025
(51) Int. Cl.: A61B 5/00

(54) **DIAGNOSTIC CAP AND METHOD USING THE SAME FOR MARKING DISTINCT AREAS OF THE SCALP**

(30) Priority: 28.05.2025 HU 2500181
(71) Applicant: Denza Hair Kft., 1133 Budapest (HU)
(72) Inventor: PÁKOZDI, Viktória, Pilisjászfalu (HU); HIMICS, Veronika, Budapest (HU); BRÁZ, Alexandra, Egercsehi (HU)
(74) Representative: Danubia Patent & Law Office LLC

(57) **Abstract**

Diagnostic cap for repeated marking of selected areas of the scalp, having a convex body following the shape of the head and comprising two straps (14, 15) and elongated ear slots (25, 26) formed at the location of the two ears, the cap is provided with a nose band (27) in the middle of the front edge (11) extending above the forehead perpendicular to its center direction, which ends after the tip of the nose, and is provided with a scale including possible positions of the tip of the nose for people of different sizes, and is provided with holes (40) in places on the head and corresponding to the diagnostic needs of the user, the size of which is between 3 and 8 mm.

During the method marking eyes (38) are placed in each area to be marked, after positioning the diagnostic cap, the tips of the marking eyes (38) are marked by touch and the cap is perforated at these locations, and before the next examination, the cap (10) is placed again and the scalp is marked through the holes (40), and the diagnostic examinations are performed in the marked areas.

## Description

The invention relates to a diagnostic cap and a method for marking discrete areas of the scalp for periodic diagnostic examinations.

It is a well-known fact that the scalp, especially the areas covered by hair, can and do differ in many properties, which can be attributed to many (sometimes unknown) reasons. Some of the characteristics of a given area can be determined by optical examinations, for example with the help of special magnification and image evaluation programs, and the current state can be stored and categorized. The results of therapeutic treatments performed at different times can be compared with each other based on such examinations, provided that it can be ensured that the individual examinations come from the same areas of the scalp. Based on the evaluation of the results of successive scalp examinations, objective statements can be made about the condition of given areas of the scalp, the changes that have occurred in them, and information can be obtained about the nature of further treatment and the method of treatment to be applied.

In order to ensure that the results of the optical examinations outlined here are comparable, it is of paramount importance that each examination is always performed on the same areas of the scalp. This is quite difficult to ensure, because the biological state of the scalp is highly area-dependent, and small local shifts can lead to results that make the comparison difficult, and marking areas that last for a long time, sometimes 3-6 weeks, is essentially an impossible requirement.

Although periodic examination of the condition of discrete areas of the scalp may be necessary for many reasons, this is of particular importance when performing special laser hair regrowth treatments, where the parameters of the treatment and the use of skin care products and medications, and/or vitamins and/or dietary supplements to be used between each treatment are significantly dependent on the results of the examinations.

UA 143544 describes a method for stimulating hair growth, during which a treatment is performed with known, but used for a different purpose, needle-like laser beams, which must be repeated if necessary, and this treatment is, according to the publication, effective and enhances hair growth. The publication presents examples of treatment with the M22 ResurFX fiber optic laser from Lumenis, where 15-30 mJ of energy is emitted at a wavelength of 1565 nm in such a way that this energy is distributed into needle beams of 50-250 pieces/cm² density.

The results and effectiveness of discrete needle laser treatments depend largely on the condition of the scalp of the treated person before the treatment, the density of the needle beams used, the energy of the pulses, the time elapsed between each treatment, and the properties of the skin care products used between each intervention. The treatment parameters to be used must be determined individually depending on the optically identifiable and categorizable condition parameters characteristic of the scalp in the given treated skin area.

In WO 2004/041325, a device used for sleep studies is to be fixed on the head, and for this purpose a mask is used, which includes straps placed on the head at different angles. Here, the mask must be placed precisely, but it is not necessary that different placements at different times are placed on the same areas on all parts of the head, and there is no information in the publication on how to ensure this.

The aim of the invention is to create a diagnostic cap whose points, some of which are distant from each other, fall on exactly the same parts of the head, and this condition can be ensured even when worn at different times.

The invention also aims to create a method for performing optical examinations of identical treated areas at different times during laser hair treatment, more precisely for repeatedly marking the same places on the scalp, sometimes at intervals of several weeks.

The set objective can be achieved by creating a diagnostic cap according to the attached claims and by performing the steps defined in the method claims.

The invention will be described in more detail below with reference to examples and with reference to the drawing. In the drawing:

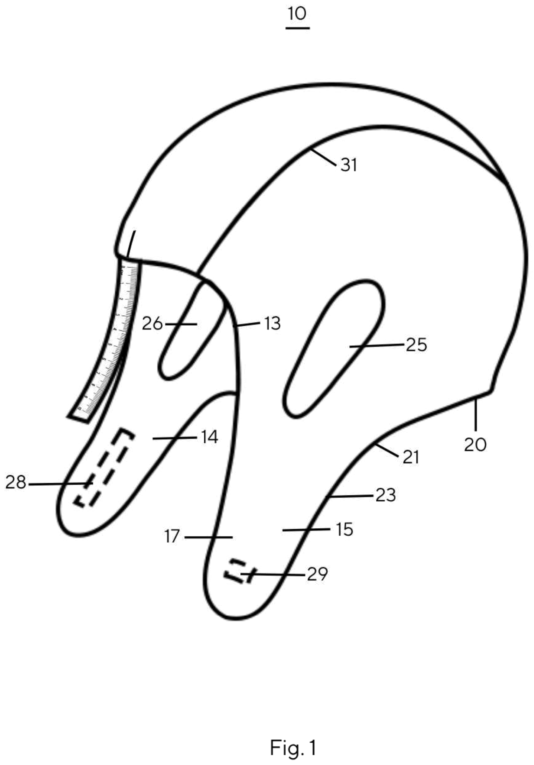

Figure 1 is a perspective view of an embodiment of the diagnostic cap according to the invention;
Figure 1 is a front view of the diagnostic cap shown in Figure 1;
Figure 1 is a rear view of the diagnostic cap shown in Figure 1;
Figure 1 shows a side view of the diagnostic cap when the position is set;
Figure 1 shows a side view of the diagnostic cap when the position is adjusted;
Figure 1 illustrates the marking of discrete selected areas;
Figure 1 shows two views of the marking eye used for marking, in distorted scale;
Figure 1 is a rear view of the diagnostic cap with the discrete locations punched out; and Figure 1 is a sketch illustrating the marking.

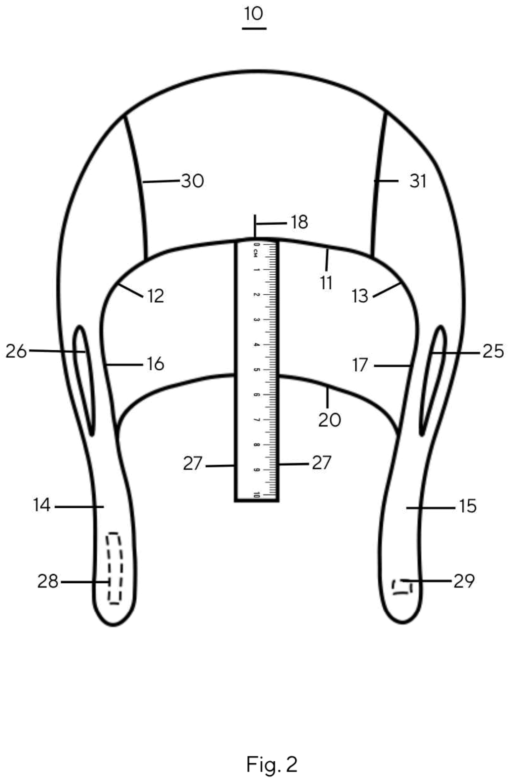

In Figures 1-3, diagnostic cap 10 according to the invention is shown in its initial state, i.e. without holes marking the areas to be examined. The word "cap" is used in a general sense, meaning any headgear that fits the head, including various helmets, masks, hoods, etc., which closely follow the shape of the head and cover the hair-covered surface of the head. The body of the diagnostic cap 10 encloses an inner space, the boundary line of which is front edge 11 outlined in Figure 2, which falls on the upper part of the forehead, slightly beyond where the area covered by hair ends in front in people with intact hair, left and right front bends 12, 13, which form a transition towards the neck through a curved arc, and on both sides there are inner edges 16, 17 of straps 14, 15 extending downwards towards the neck and thinning in the direction thereof , which are also slightly curved, but their radius of curvature increases as they go downwards.

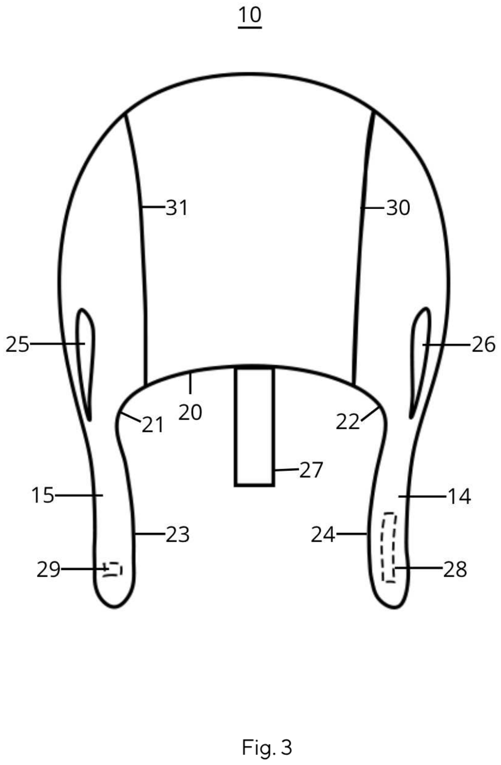

The rear view of Figure 3 shows the rear edge 20 of the diagnostic cap 10, the longest section of which is slightly curved, convex when viewed downwards, and follows the boundary line of the nape and neck and is connected laterally to the rear edges 23, 24 of the two straps 14, 15 through two curved sections 21, 22, and then at their lower ends they bend forward along a strong curvature and are connected to the inner edges 16, 17 of the straps 14, 15. The edges thus shown are spatial curved lines between which the almost completely continuous, convex body of the diagnostic cap 10 can be interpreted when viewed from above. The continuity is interrupted only by ear slits 25, 26 formed at the corresponding locations for the two ears, the width of which is almost the same as the external size of the earmold, and therefore, when the diagnostic cap 10 is put on, its lateral position on the head is determined quite accurately.

In front, the middle of the front edge 11 is preferably marked by a short center line 18 produced by sewing or coloring, and symmetrically thereto, a noseband 27 is provided with length marking lines (e.g., the cm and mm graduation lines also used on measuring tapes), the length of which is sufficiently long to extend beyond the tip of the wearer's nose and to enable the location of the tip of the nose to be marked. Although the drawing shows the graduation lines along the entire length of the noseband 27, it may be sufficient if such marking is only provided in the range of possible nose tip locations for different individuals.

In the region of the straps 14, 15 opposite each other, overlapping each other, falling under the chin, it is advisable to form a section corresponding to a Velcro fastener, by means of which the straps 14, 15 can be connected to each other and disassembled. In the possible region of connection, an elongated marking strip 28 is applied to one of the straps, in the example the right-hand strap 14, and a small marking point 29 is applied to the other strap 15, but a different marking can also be used.

Due to the spatial shape of the diagnostic cap 10, it cannot be made from a single flat material, but is assembled by sewing from several, sometimes appropriately pre-shaped, parts, and in the drawing, two main seam lines 30, 31 are depicted in order to illustrate the spatial design, which, if clearly visible, help to put the diagnostic cap 10 on correctly.

The material of the diagnostic cap 10 is preferably a flexible, elastic, double-knit (interlock) synthetic fiber fabric, a typical example of which is called " scuba" in the technical language.. Its material is similar to the fabric commercially available under the name " Neoprene ^{™}", whose chemical name is " polychloroprene ".

This material is characterized by a smooth, slightly spongy feel, and is slightly lighter and more flexible than the Neoprene ^{™} fabrics mentioned, making it much more versatile. There are many more reviews of this material on the internet.

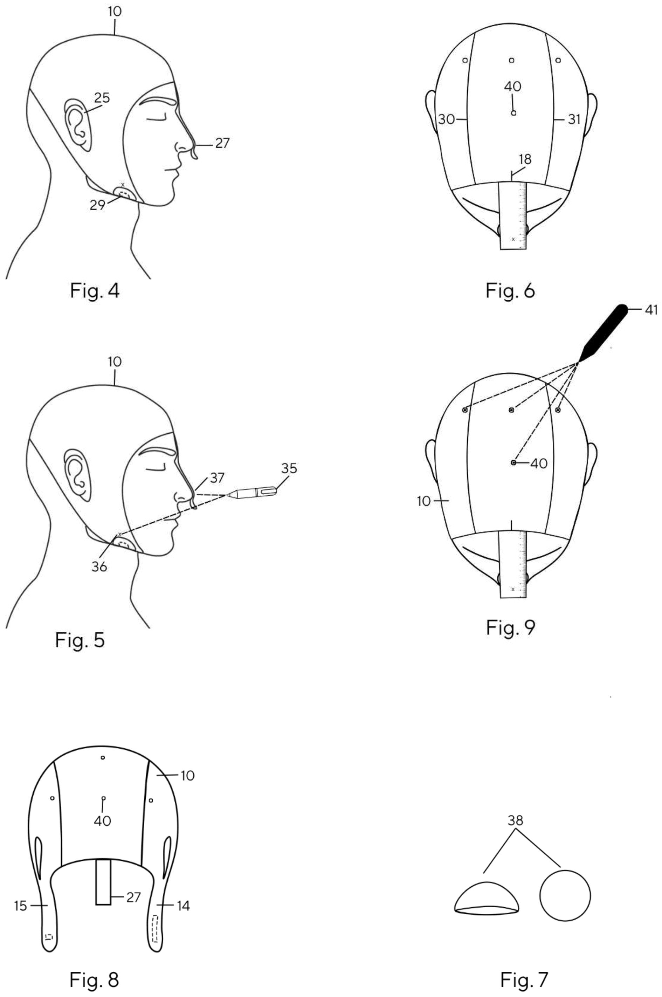

We now refer to Figures 4 and 5, which illustrate the position fixing of the diagnostic cap 10 when it is first put on. For the sake of simplicity, the seam lines 30, 31 have not been shown here. A set of sufficiently accurate steps according to head size is made from the diagnostic caps 10 described so far, from which the copy that fits the head size of the given person is selected according to the length of the head circumference, and then placed on the head, we obtain the position outlined in Figures 4 and 5, when the straps 14, 15 are placed overlapping each other so that they fit sufficiently tightly under the chin, but not yet disturbing the person being examined. After that, using a marking pen 35 outlined in Figure 5, we mark the location 36 of the end of the upper strap 15, then we record which point 37 of the length scale on the noseband 27 the tip of the nose falls on, and we record this so that we can take it into account during subsequent examinations of the person concerned..

The position of the diagnostic cap 10 on the head is precisely determined by several positioning elements. These include the correct selection of the size, then the fitting of the ears into the two ear slots 25, 26, which together with the fitting of the center line 18 to the center line of the head determines the lateral position, in addition to these, it is important to identify the position of the tip of the nose in the forward-backward direction, and finally the proper fit and support is achieved by adjusting the tension of the straps 14, 15. This orientation can ensure that the position of the diagnostic cap 10 and the head is the same at every use.

The method of diagnostic use is now described in connection with Figures 6 and 7. The purpose of the examination can be diverse, but here we refer primarily to the use for laser hair treatment. Such treatment is preceded by a professional examination of the scalp and hair density of the examined person, as well as the degree of hair loss, and based on this, characteristic areas of the scalp that differ from each other in some parameter are determined, in the range of which the treatment and/or the care between treatments must be performed in respective different ways. The determination of the treatment is not the subject of the application. According to experience, the number of places to be marked from such different regions is usually between 3 and 10, depending on the person in question. In the middle of these regions, hemispherical marking eyes 38, outlined in both directions in Figure 7, with a flat surface coated with a material that ensures adhesion, are placed, the diameter of which is typically between 3 and 8 mm, preferably 5 mm. After the marking eyes 38 have been placed on he selected areas, the diagnostic cap 10 is placed on the head of the person being examined. The adhesion of the marking eyes 38 is strong enough to maintain their position when the diagnostic cap 10 is put on. At the peak points of the protrusions of the marking eyes 38, the material of the diagnostic cap 10 is marked from the outside with a marking pen, and then the material of the diagnostic cap 10 is perforated at these marked locations with a punching tool. The material of the fabric referred to is strong and stable enough that the edges of the holes between 3-8 mm remain intact over time; when a different cap material is chosen, the position of the holes can be stabilized by known hole edging solutions. Figure 6 shows the formation of four such holes 40 on the head in a top view, and the same is seen in Figure 8 in a rear view of the diagnostic cap 10 removed from the head.

During periodic use of the diagnostic cap 10, for example 3-5 weeks after the first treatment, it is placed on the head, then the respective centers of the regions to be examined are clearly marked on the scalp through the holes 40 with the marking device 41 schematically depicted in Figure 9, and the optical examinations are performed in the regions with the centers corresponding to the markings. Since the effect of a hair treatment process takes a long time, more such intermediate examinations are needed, and based on the results of such examinations it can be determined whether the laser treatment needs to be repeated, and if so, under what parameters, and what skin care or even medication is needed between treatments.

By using the diagnostic cap 10 according to the invention, the centers of different regions of the scalp from each other in terms of examination for a diagnostic purpose can be accurately identified even if a long time has passed between the subsequent individual examinations. It is advisable to store the diagnostic cap 10 between examinations either with the examined person or at the treatment site, protected from external influences, assigned to the given person.

## Claims

1. Diagnostic cap for repeated marking of selected areas of the scalp of a subject, made of flexible, synthetic fiber material, having a convex body following the shape of the head, the edges of which are defined by a curved line, the front edge (11) of which falls on the upper part of the forehead, then it has front bends (12, 13) on the left and right sides, which form a transition towards the neck through a curved arc, and on both sides said cap has inner edges (16, 17) of straps (14, 15) extending downwards towards the neck and tapering in the direction thereof, the radius of curvature of said edges (16, 17) increases in downward direction, and the straps (14, 15) have rear edges (23, 24) curved backwards in an arc, the upper ends of which close the line of said rear edges through a further rear edge (20) falling in the line of the nape, and said cap (10) also includes elongated holes formed at the location of the two ears ear slits (25, 26) **characterized in that** the front edge (11) is provided with a nose strip (27) in the middle of the front edge (11) perpendicular to the center thereof, which ends after the tip of the nose, and is provided with a scale including possible nose tip positions for subjects having different sizes, and the cap is provided with holes (40) in places on the head corresponding to the diagnostic needs of the user, the size of which being between 3 and 8 mm.

2. The diagnostic cap (10) according to claim 1, wherein the straps (14, 15) each have a Velcro region in the area under the chin, and the connected position of the straps (14, 15) on the person being examined is fixed by a visibly marked location (36) on one of the straps.

3. The diagnostic cap (10) according to claim 1 or 2, wherein the noseband (27) has a center line (18) indicating the center of the cap (10) at the base.

4. The diagnostic cap as claimed in any one of claims 1 to 3, wherein the material thereof is a fabric containing double-bonded polychloroprene fibers.

5. The diagnostic cap as claimed in any one of claims 1-4, wherein having visible seam lines (30, 31) arranged symmetrically to the center on both sides.

6. Method for marking discrete areas of the scalp for periodic diagnostic examinations using the diagnostic cap (10) as claimed in any one of claims 1 to 5, which is initially not provided with holes (40), **characterized in that** in a first step, placing marking eyes (38) with tactile tips adhering to the scalp on characteristic separate areas on the head of a subject that are significant for diagnostic examinations, then placing the diagnostic cap (10) on the head and marking the peaks of the marking eyes (38) under the diagnostic cap (10) by touching said peaks, and forming respective said holes (40) around these marked peaks, and when the result of the treatment performed on the given person needs to be checked after some time has elapsed since the holes are made, placing the diagnostic cap (10) on the head of the subject in the same position, and marking the scalp through the holes, and after removing the diagnostic cap (10) performing diagnostic tests around said marked areas, and if the diagnostic tests need to be repeated one or more times after respective periods, repeating said markings and carrying out the required diagnostic tests.

7. The method according to claim 6, wherein the marking eyes (38) are hemispherical and have a diameter of between 3 and 8 mm.

8. The method according to claim 6 or 7, wherein respective intervals of 3-8 weeks being left between each of said repetitions.

9. The method as claimed in any one of claims 6-8, wherein during the diagnostic examinations subjecting the marked areas to instrumental optical inspections.
